# EUROPEAN PATENT SPECIFICATION

(11) **EP 3 453 376 B1**
(45) Date of publication and mention of the grant of the patent: **30.03.2022**
(21) Application number: 17792674.8
(22) Date of filing: 13.04.2017
(51) Int. Cl.: A61M 5/00, A61J 1/16, A61J 1/06, B65D 55/04

(54) **SYRINGE HOLDER AND SYRINGE STORAGE CONTAINER PROVIDED WITH SAME**
SPRITZENHALTER UND DAMIT AUSGESTATTETES SPRITZENAUFBEWAHRUNGSBEHÄLTNIS
SUPPORT DE SERINGUE ET RÉCIPIENT DE STOCKAGE DE SERINGUE POURVU DE CELUI-CI

(30) Priority: 02.05.2016 JP 2016092631
(43) Date of publication of application: 13.03.2019
(73) Proprietor: Taisei Kako Co., Ltd., Kita-ku Osaka-shi Osaka 531-0072 (JP)
(72) Inventor: SONOYAMA, Tomoyuki, Ibaraki-shi Osaka 567-0054 (JP); KATAGIRI, Yuki, Ibaraki-shi Osaka 567-0054 (JP)
(74) Representative: Stolmár & Partner Patentanwälte PartG mbB
(86) International application number: PCT/JP2017/015044
(87) International publication number: WO 2017/191741

(56) References cited:
- EP-A1- 2 119 463
- EP-A1- 2 865 400
- WO-A1-2008/107961
- WO-A1-2013/031264
- US-A1- 2014 014 654

## Description

### CROSS-REFERENCE TO RELATED APPLICATION

This application claims priority to Japanese Patent Application No. 2016-092631 filed on May 2, 2016.

### FIELD OF THE INVENTION

The present invention relates to a syringe holder that holds syringes and a syringe storage container that includes the syringe holder.

### BACKGROUND ART

When syringes are transported from a factory where the syringes are manufactured to a factory where the syringes are filled with medicine for filling empty syringes with medicine, a syringe storage container 100 as shown in Fig. 6 is used, for example (Patent Literature 1).

This syringe storage container 100 includes a container body 101 and a fitting plate 102 that serves as a syringe holder to hold a syringe T. The fitting plate 102 includes a base 103 that is provided with insertion through holes through which the syringes T are inserted, and receiving cylinders 104 in a cylindrical shape that protrude respectively from opening end edges of the insertion through holes. Each of the receiving cylinders 104 is configured to lock a flange T1 of the syringe T at an opening end edge 105, when the syringe T has been inserted through the receiving cylinder 104, to thereby hold the syringe T. The syringe storage container 100 is sealed with a protective film and transported while the fitting plate 102 with the syringes T held therein is stored in the container body 101.

### CITATION LIST

Patent Literature 1: WO 2014/049712 A1

The fitting plate 102 made of polypropylene (PP) is often used for the aforementioned conventional syringe storage container 100. Such a fitting plate 102 made from polypropylene (PP) is too hard for the syringe T made from cyclic polyolefin (COP) because it easily scratches the syringe T. Therefore, there has been a problem that, when the syringe T made from cyclic polyolefin (COP) is held in the fitting plate 102, the syringe T during the transport moves within the receiving cylinder 104, rubs against an inner wall surface 106 of the receiving cylinder 104, and thereby gets scratched.

In order to deal with this problem, it is conceivable to use a resin with low hardness as a material for the fitting plate 102, which may not easily cause scratches on the syringe T made from cyclic polyolefin (COP). However, such a resin with low hardness has a problem of causing a poor strength of the fitting plate 102 itself.

US 2014/014654 A1 relates to a carrier plate for storing/ transporting pharmaceutical containers.

EP 2 865 400 A1 relates to a syringe storage container allowing a syringe to be pulled out therefrom without raising a syringe holding section.

### SUMMARY

### Technical Problem

In view of the aforementioned circumstances, it is an object of the present invention to provide a syringe holder that has a sufficient strength and does not easily cause scratches on a syringe, and a syringe storage container that includes the syringe holder.

### Solution to Problem

The syringe holder according to the present invention is a syringe holder for holding syringes including: a base that is provided with a plurality of insertion through holes through which syringes are inserted; a plurality of wall parts having a cylindrical shape that project respectively from opening end edges of the plurality of insertion through holes of the base, the wall parts having an inner diameter that is the same as a diameter of the insertion through holes; and ribs, each of which connects each adjacent ones of the plurality of wall parts, wherein the ribs have a height from the base of 40% or more and 100% or less of a height from the base of the wall parts, the wall parts have an inner diameter of 100% or more and 115% or less of an outer diameter of barrels of the syringes, and at least an inner wall surface of each of the wall parts is made from a material including polyethylene as a main component.

As one embodiment of the present invention, the ribs may have a height from the base of 50.0% or more and 100% or less of a height from the base of the wall parts.

The syringe storage container according to the present invention includes the aforementioned syringe holder and a container body that stores the syringe holder.

### BRIEF DESCRIPTION OF THE DRAWINGS

Fig. 1 is an appearance view of a syringe storage container according to one embodiment of the present invention.
Fig. 2 is a front view of a syringe holder in the syringe storage container according to the embodiment.
Fig. 3 is a cross-sectional view of the syringe holder according to the embodiment, taken along the line III-III in Fig. 2.

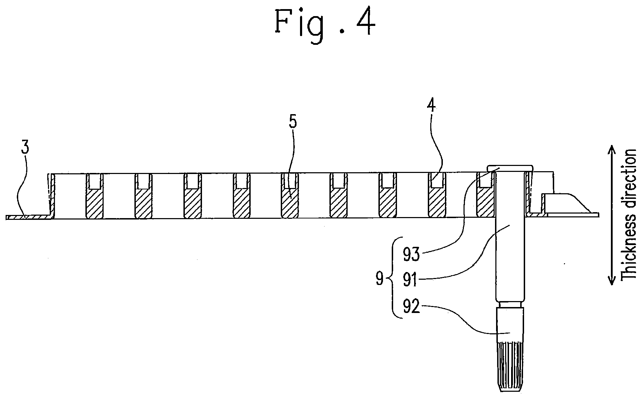

Fig. 4 is a cross-sectional view of the syringe holder according to the embodiment, taken along the line IV-IV in Fig. 2.
Fig. 5 is an enlarged cross-sectional view of a portion of the syringe holder of Fig. 2 where a syringe is inserted therethrough, according to the embodiment.
Fig. 6 is an appearance view of a conventional syringe storage container.
Fig. 7 is a cross-sectional view of a fitting plate of the conventional syringe storage container in a deflected state, taken along the line VII-VII in Fig. 6.
Fig. 8 is a cross-sectional view of the conventional fitting plate in a deflected state, taken along the line VIII-VIII in Fig. 6.
Fig. 9 is an enlarged cross-sectional view of a portion of the conventional fitting plate of Fig. 7 where a syringe is inserted therethrough in a deflected state.

### DESCRIPTION OF EMBODIMENTS

There is a great demand for a syringe made from cyclic polyolefin because the syringe made from cyclic polyolefin has properties such as high transparency and excellent strength. Therefore, it is strongly desired to provide a syringe holder that can hardly scratch a syringe made from cyclic polyolefin during the transport. With an approach from both perspectives in the material and the strength of the syringe holder, this embodiment provides a syringe holder that has sufficient strength and does not easily scratch a syringe, and a syringe storage container that includes the syringe holder.

Hereinafter, a syringe storage container according to one embodiment of the present invention will be described with reference to the drawings.

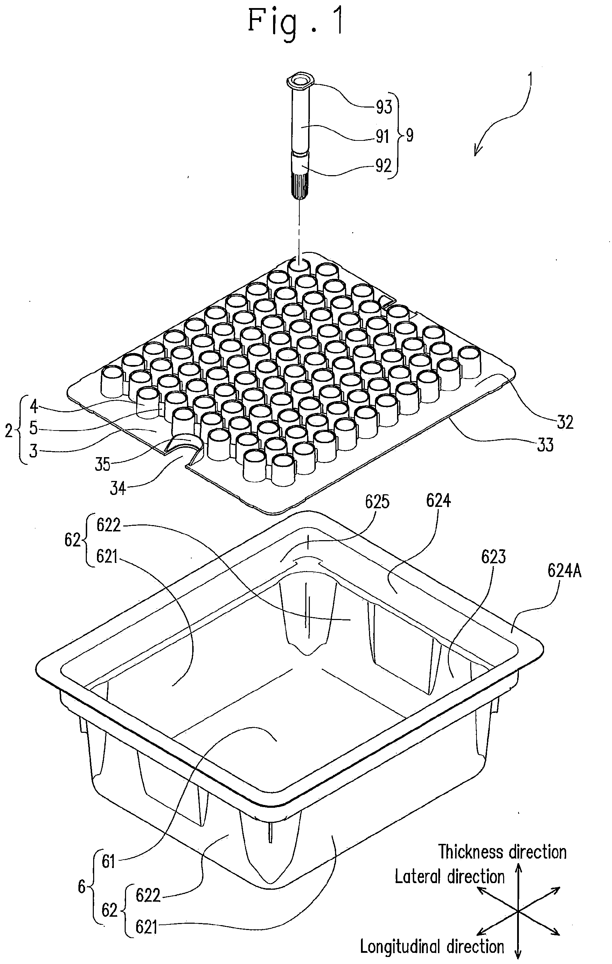

As shown in Fig. 1, each of syringes 9 to be stored in a syringe storage container 1 of this embodiment includes a barrel 91 in a cylindrical shape, an injection needle (not shown) that is fixed to a distal end of the barrel 91, a cap 92 that covers the injection needle, and a flange 93 that is provided on the proximal end side of the barrel 91 to extend along an outer circumference of the barrel 91. The barrel 91 of the syringe 9 is formed of a cyclic polyolefin resin.

The syringe storage container 1 according to this embodiment includes a syringe holder 2 that holds the syringes 9 and a container body 6 that stores the syringe holder 2.

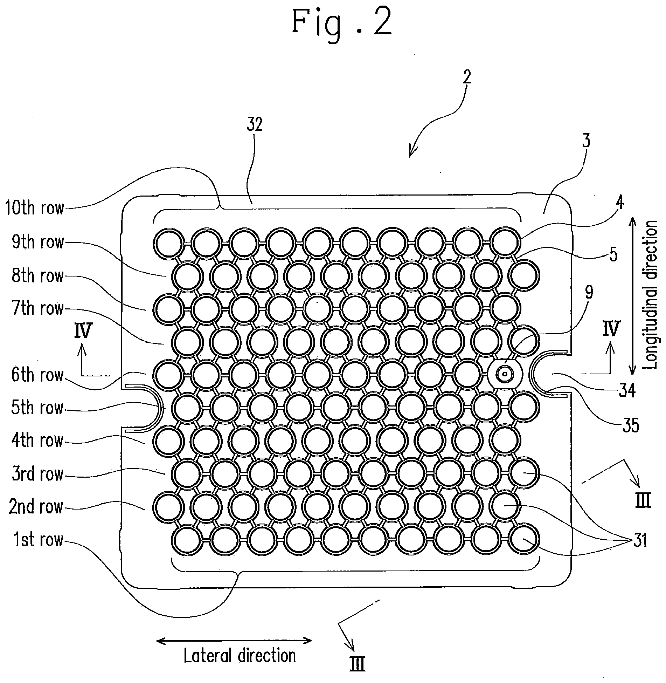

As shown in Fig. 1 and Fig. 2, the syringe holder 2 includes a base 3 that supports the syringes 9, wall parts 4 in a cylindrical shape, each of which holds the syringe 9 inserted therethrough, and ribs 5, each of which connects each adjacent ones of the wall parts 4 with each other. The syringe holder 2 of this embodiment is made from polyethylene.

The base 3 is provided with a plurality of insertion through holes 31 through which the syringes 9 are respectively inserted. In this embodiment, the base 3 is provided with one hundred insertion through holes 31. The base 3 is formed into a plate shape and provided with insertion through holes 31 to pass through the base 3 in the thickness direction. The base 3 of this embodiment is formed into a thin rectangular plate shape having long sides and short sides. Herein, the direction in which the long sides extend is referred to as the lateral direction, and the direction in which the short sides extend is referred to as the longitudinal direction. Ten insertion through holes 31 are formed to align along the lateral direction of the base 3, and ten rows each having ten insertion through holes 31 are aligned in the longitudinal direction to thereby provide one hundred insertion through holes 31.

The base 3 has a front side 32 and a rear side 33 and is mounted on a container body 6 with the rear side 33 facing a bottom part 61 to be described below. The base 3 is provided with a pair of cutouts 34 on the opposite sides. The syringe holder 2 can be mounted on the container body 6 or taken out from the container body 6 by the engagement of fingers or hooks with the rear side 33 of the base 3 through the cutouts 34. The base 3 has extensions 35 that extend respectively from the peripheral edges of the cutouts 34 on the front side 32 side. The cutouts 34 are formed on the short sides in this embodiment.

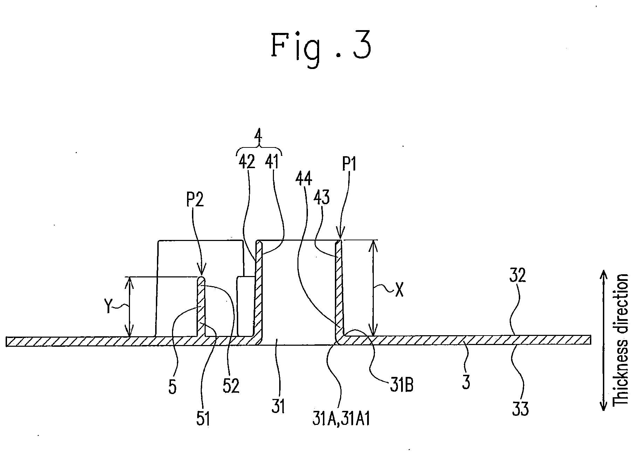

As shown in Fig. 3, an inner circumference surface 31A of each of the insertion through holes 31 is configured to increase in diameter toward the rear side 33 side of the base 3. The inner circumference surface 31A of the insertion through hole 31 of this embodiment serves as a curved portion 31A1 forming a curved surface. The inner circumference surface 31A of the insertion through hole 31 continues smoothly without roughness to an inner wall surface 41 of the wall part 4 to be described below.

Turning to Fig. 2, each of the insertion through holes 31 has a circular opening as shown therein. In this embodiment, all of the insertion through holes 31 are formed to have the same diameter. The insertion through holes 31 adjacent to each other in the lateral direction are formed to have a fixed distance therebetween. The row of the insertion through holes 31 located on the lowermost side of the paper surface in Fig. 2 is referred to as the 1st row, and the subsequent rows of the insertion through holes 31 toward the upper side in the longitudinal direction of the paper surface are regarded to align respectively as the 2nd row, the 3rd row, ... in order. In this embodiment, the insertion through hole 31 located at the left end of the 2nd row is arranged closer to the left side than the insertion through hole 31 located at the left end of the 1st row. Specifically, the left end of the 2nd row projects to the left side from the left end of the 1st row by a half distance that equivalents to half of the distance between the insertion through holes 31 adjacent to each other in the lateral direction.

In this embodiment, the left ends of the even rows of the 1st row to the 10th row project more to the left side by the half distance than the left ends of the odd rows. That is, the right ends of the odd rows project more to the right side by the half distance than the right ends of the even rows. In this embodiment, the insertion through holes 31 are aligned in a zigzag alignment (alignment in a hexagonal lattice) so that each one of the insertion through holes 31 is surrounded by six insertion through holes 31. The distances between the adjacent insertion through holes 31 are equal to each other in all the radially aligned insertion through holes 31.

The wall parts 4 are formed into a cylindrical shape having a certain thickness. The wall parts 4 of this embodiment are formed into a circular cylindrical shape. As shown in Fig. 3, each of the wall parts 4 has an inner wall surface 41 that constitutes an inner circumference surface of the wall part 4, and an outer wall surface 42 that constitutes an outer circumference surface of the wall part 4. The wall part 4 projects from one of both sides of the base 3. The wall parts 4 of this embodiment projects from the front side 32 of the base 3. In the following description, the height X of the wall part 4 is referred to as, by taking the uppermost projecting portion in the distal end 43 of the wall part 4 as a peak P1 with the front side 32 of the base 3 as a reference, the length from the front side 32 of the base 3 to the peak P1 of the wall part 4. The peak P1 of this embodiment is a distal end of the outer wall surface 42 of the wall part 4.

The wall part 4 of this embodiment projects from the front side 32 of the base 3 so as to have the thickness direction of the base 3 matched with the axial direction of the wall part 4. The wall part 4 projects from an opening end edge 31B of the insertion through hole 31 formed in the base 3.

The wall part 4 of this embodiment is formed to have a substantially uniform inner diameter from a proximal end 44 located on the base 3 side to a distal end 43 on the side, from which the syringe 9 is inserted through. In this embodiment, the distal end portion of the wall part 4 is configured to increase in inner diameter toward the distal end 43. Specifically, the inner wall surface 41 is chamfered at the distal end 43 along the circumferential direction to form an inclined surface that inclines toward the peak P1. The wall part 4 is formed to have a uniform inner diameter as the inner diameter of the insertion through hole 31 of the base 3. The wall part 4 of this embodiment is formed to decrease (narrow) in outer diameter from the proximal end 44 toward the distal end 43. Specifically, the thickness of the wall part 4 decreases from the proximal end 44 toward the distal end 43. That is, the inner wall surface 41 is formed straight so as to be parallel with the axial direction in a portion other than the distal end portion, while the outer wall surface 42 is formed to incline inward in the radial direction toward the distal end 43.

The inner diameter of the wall part 4 is larger than the outer diameter of the cap 92 and the outer diameter of the barrel 91 of the syringe 9, and smaller than the outer diameter of the flange 93 of the syringe 9. The wall part 4 can support the flange 93 of the syringe 9 at the distal end of 43 of the wall part 4 while the syringe 9 is held inserted through the wall part 4. The wall part 4 is formed to have an inner diameter of 100% or more and 115% or less of the outer diameter of the barrel 91 of the syringe 9. Preferably, the wall part 4 is formed to have an inner diameter of 100% or more and 105% or less of the outer diameter of the barrel 91 of the syringe 9.

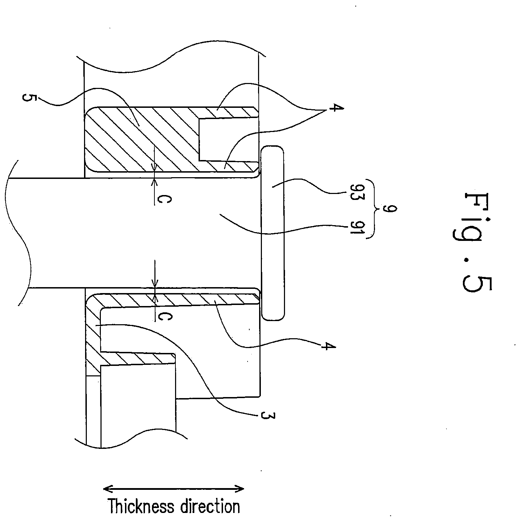

The wall part 4 with the inner diameter of 100% or more and 115% or less of the outer diameter of the barrel 91 of the syringe 9 allows the barrel 91 of the syringe 9 to insert through the wall part 4 and prevent excessive enlargement of the clearance C (see Fig. 5) between the wall part 4 and the barrel 91 of the syringe 9. That is, this is because the wall part 4 is made to suppress the movement of the syringe 9 within the wall part 4 to thereby hardly cause scratches on the syringe 9. The wall part 4 with the inner diameter of 105% or less of the outer diameter of the barrel 91 of the syringe 9 can further reduce scratches on the syringe 9.

The height X of the wall part 4 in this embodiment is set to be greater than the outer diameter of the wall part 4. The thickness of the wall part 4 in this embodiment is set to be smaller than the thickness of the base 3 and the ribs 5.

A plurality of wall parts 4 are provided on the base 3. In this embodiment, the wall parts 4 of a number corresponding to the number of the insertion through holes 31 of the base 3 are provided. That is, each of the wall parts 4 projects from an opening end edge 31B of each of the insertion through holes 31. In the following description, as to the alignment of the wall parts 4, the row of the wall parts 4 located on the lowermost side of the paper surface in Fig. 2 is referred to as the 1st row, and the subsequent rows of the wall parts 4 toward the upper side in the longitudinal direction of the paper surface are regarded to align respectively as the 2nd row, the 3rd row, ... in order, in the same manner as the alignment of the insertion through holes 31 of the base 3. As to the height X of the wall parts 4, all of the wall parts 4 have the same height in this embodiment.

As shown in Fig. 1 and Fig. 2, each of the ribs 5 is a part to connect the wall parts 4 with each other. Each of the ribs 5 of this embodiment connects each adjacent ones of the wall parts 4. The ribs 5 of this embodiment are formed into a plate shape having a certain thickness. Specifically, each of the ribs 5 is a plate member having long sides and short sides.

As shown in Fig. 3, the ribs 5 project from one of both sides of the base 3. The ribs 5 of this embodiment project from the front side 32 of the base 3. The ribs 5 project from the front side 32 of the base 3 to have the thickness direction of the base 3 matched with the longitudinal direction of the ribs 5. One of the pair of the short sides of the rib 5 is a proximal end 51 located on the base 3 side, and the other one of the short sides is a distal end 52 located on the distal end 43 side of the wall part 4. That is, the rib 5 has the long sides through which the wall parts 4 are connected with each other. In the following description, the height Y of the rib 5 is referred to, by taking the uppermost end of a coupling portion of the rib 5 with the wall part 4 as a peak P2 with the front side 32 of the base 3 as a reference, the length from the front side 32 of the base 3 to the peak P2 of the rib 5. That is, it is assumed that the strength of the syringe holder 2 is influenced by the height of the portion at which the rib 5 and the wall part 4 are coupled with each other, and thus, even if the rib 5 is high, the influence to the strength of the syringe holder 2 is small unless the rib 5 is coupled with the wall part 4. The peak P2 of this embodiment is a distal end surface of the distal end 52 of the rib 5.

The rib 5 of this embodiment is formed to be thicker on the proximal end 51 side than on the distal end 52 side. Specifically, the rib 5 is formed into a tapered shape having its sectional shape in the thickness direction tapered toward the distal end 52. The rib 5 is formed so that the thickness of the thickest part in the area on the proximal end 51 side is smaller than the thickness of the base 3. The distal end 52 of the rib 5 is chamfered to have a curved shape.

The rib 5 is configured to have the height Y from the base 3 of 40% or more and 100% or less of the height X of the wall part 4 from the base 3. The rib 5 of this embodiment is configured to have the height Y from the base 3 of 50.0% or more and 100% or less of the height X of the wall part 4 from the base 3.

The rib 5 is configured to have the height Y of 40% or more of the height X of the wall part 4 from the base 3 because the rib 5 of the height Y lower than 40% of the height X of the wall part 4 deteriorates the effect of suppressing the deflection of the base 3, and hence reduce the strength of the syringe holder 2. The rib 5 is configured to have the height Y of 100% or less of the height X from the base 3 of the wall part 4 (that is, the rib 5 is as high as the wall part 4, or lower than the wall part 4) because the upper limit of the portion at which the rib 5 and the wall part 4 are coupled with each other is 100%.

As shown in Fig. 2, in this embodiment, the ribs 5 are arranged so as to provide an mixed arrangement of the wall parts 4 with the ribs 5 connected therebetween and the wall parts 4 without the ribs 5 connected therebetween. Specifically, the ribs 5 are arranged to connect each adjacent ones of the wall parts 4 aligned in certain rows and, while not connecting each adjacent ones of the wall parts 4 aligned in the other certain rows. In this embodiment, the wall parts 4 aligned in the 2nd row, the 4th row, the 7th row, and the 9th row are not connected by the ribs 5 in the lateral direction, while connected by the ribs 5 in the other directions.

As shown in Fig. 1, the container body 6 is a bottomed container. The container body 6 of this embodiment includes a bottom part 61 having a rectangular plate shape and a sidewall 62 extending from a peripheral edge of the bottom part 61. The bottom part 61 is a plate member having long sides and short sides. The sidewall 62 includes a pair of lateral sidewalls 621 opposite to each other in the short side direction (longitudinal direction) and a pair of longitudinal sidewalls 622 opposite to each other in the long side direction (lateral direction). Both ends of the pair of lateral sidewalls 621 in the long side direction and both ends of the pair of longitudinal sidewalls 622 in the short side direction are connected to each other to form the sidewall 62 extending along the peripheral edge of the bottom part 61.

The sidewall 62 continues along the peripheral edge of the bottom part 61 and includes a lower sidewall 623 that forms an opening area having substantially the same area as that of the bottom part 61 and an upper sidewall 624 that forms an opening area larger than the opening area of the lower sidewall 623. The upper sidewall 624 has an opening end edge that is formed into a flange shape and serves as a flange 624A. The sidewall 62 includes a step 625 at the boundary between the lower sidewall 623 and the upper sidewall 624 at which the opening area of the lower sidewall 623 is enlarged to the opening area of the upper sidewall 624.

The syringe storage container 1 according to this embodiment has been described above. Hereinafter, the usage of the syringe storage container 1 will be described below.

When the syringes 9 are transported using the syringe storage container 1, each syringe 9 is inserted through each wall part 4 as shown in Fig. 1 so that the syringes 9 are inserted through all of the wall parts 4. When the insertion of the syringes 9 are completed, the syringe holder 2 is lifted and mounted on the step 625 of the container body 6 so as to have the lateral direction of the base 3 matched with the longitudinal direction of the container body 6. Alternatively, the syringes 9 are inserted through the wall parts 4 while the syringe holder 2 is mounted on the step 625 of the container body 6. In such a state where the syringe holder 2 with the syringes 9 held therein is mounted in the container body 6, a protective film (not shown) formed of a resin sheet is adhered to or heat fused to the flange 624A of the upper side wall 624 so that the container body 6 is sealed. Thus, the syringes 9 are held in a transportable state.

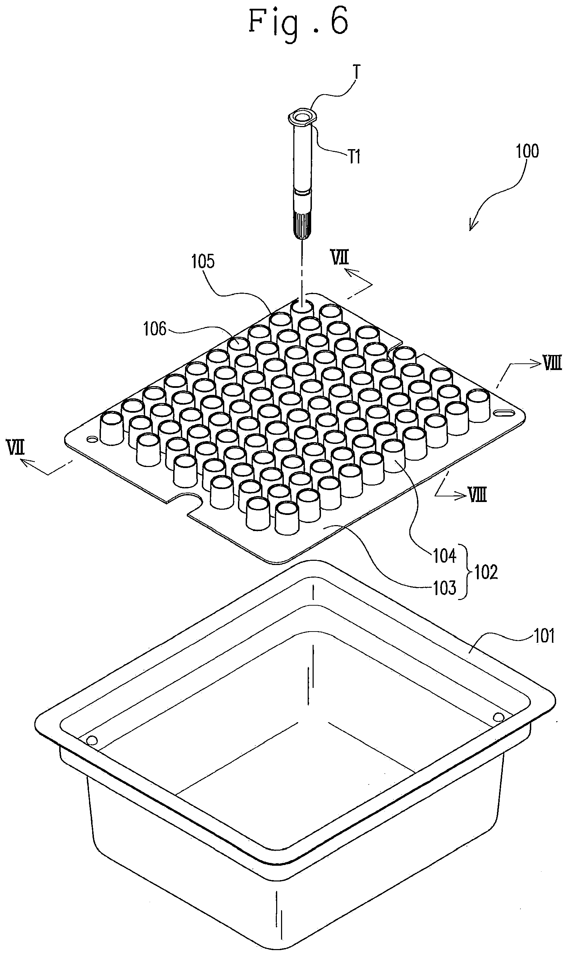

Hereinafter, the description will be provided for the relationship among the material of the syringe holder 2, the inner diameter of the wall parts 4, and the height Y of the ribs 5, which contribute to impart sufficient strength to the syringe holder 2 of the syringe storage container 1 of this embodiment and suppress the scratches on the syringe 9, with reference to the conventional syringe storage container 100 shown in Fig. 6. When the description is provided with reference to the conventional fitting plate 102, the corresponding parts and members in the aforementioned embodiment are denoted by the same reference numerals and the same names.

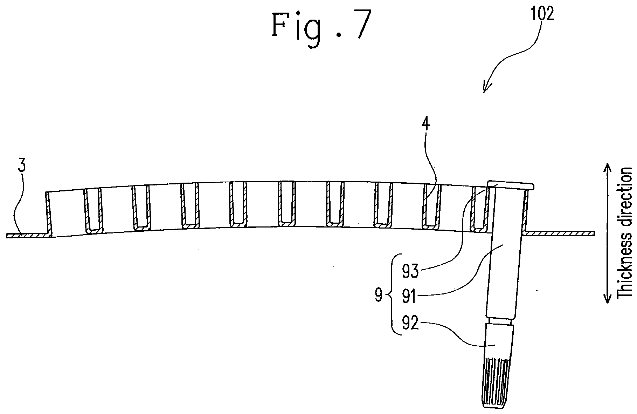

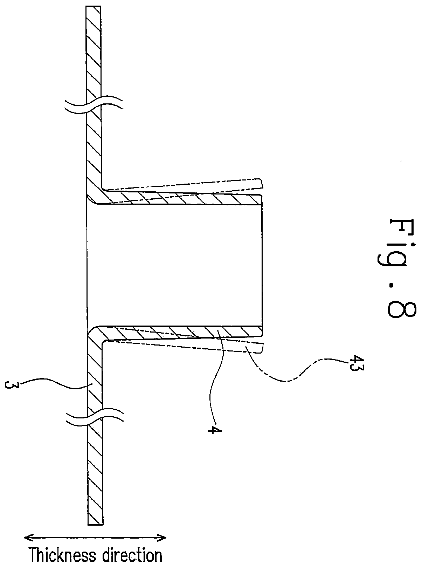

The base 3 of the fitting plate 102 is conventionally formed into a thin plate. The wall part 4 is also formed with a thin wall. Therefore, the conventional fitting plate 102 may be deformed due to the deflection of the base 3 (for example, Fig. 7) or the deformation of the wall part 4 (for example, the double-dotted line in Fig 8) in the treatment process such as the sterilization treatment or the heating treatment.

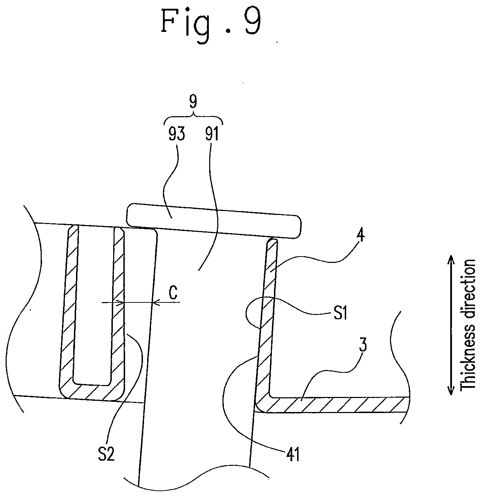

Fig. 9 is an enlarged view of the portion of Fig. 7 with the syringe 9 inserted therethrough. For example, when deflection occurs in the base 3 as shown in Fig. 7, the syringe 9 moves to the lower side in the wall part 4 as shown in Fig. 9 so that the syringe 9 is transported while the barrel 91 is held in contact with the inner wall surface 41 of the wall part 4. That is, the syringe 9 is held in a deflected state in the wall part 4, thereby causing a large clearance C between the barrel 91 of the syringe 9 and the inner wall surface 41 (S2 in Fig. 9) located on the side opposite to the abutting surface S1 at which the wall part 4 abuts the barrel 91 of the syringe 9.

When the syringe 9 is transported in the deflected state in the wall part 4, the syringe storage container 100 is shaken so that the syringe 9 rotates in the wall part 4, while largely moves in the radial direction of the wall part 4. Therefore, the syringe 9 transported using the deflected base 3 is subjected to large impact when the syringe 9 abutting the abutting surface S1 collides against the inner wall surface S2 so that the barrel 91 of the syringe 9 more easily gets scratches than the syringe 9 transported using the non-deflected base 3.

When the base 3 is deflected toward the side where the wall part 4 projects in the thickness direction as shown in Fig. 7, the insertion through hole 31 of the base 3 is radially pulled and thereby the opening end edge of the insertion through hole 31 is radially stretched, which causes the opening of the wall part 4 on the distal end 43 side as shown in Fig. 8 and Fig. 9 to be widened and hence the clearance C between the inner wall surface 41 of the wall part 4 and the barrel 91 of the syringe 9 to be enlarged. Therefore, the barrel 91 of the syringe 9 gets many scratches when the syringe 9 is transported using the deformed fitting plate 102.

Even if a hard material is used for the fitting plate 102 to provide a hardly deformable fitting plate 102, it is difficult to reduce scratches on the syringe 9 made from cyclic polyolefin due to the hardness.

The syringe storage container 1 according to the aforementioned embodiment is designed to hardly scratch the syringe 9, while ensuring the strength. In order to achieve this object, the wall part 4 has such an inner diameter that can enhance the strength of the wall part 4 itself and keep the clearance C between itself and the syringe 9 small, the wall parts 4 are connected with each other by the ribs 5 having a certain height, and a material including polyethylene as a main component is employed as the material for the inner wall surface 41 of the wall part 4.

That is, the syringe holder 2 in this embodiment is configured to have the height Y of the rib 5 of 40% or more and 100% or less of the height X of the wall part 4, and have the inner diameter of the wall part 4 of 100% or more and 115% or less of the outer diameter of the barrel 91 of the syringe 9. The wall parts 4 having an inner diameter set within the above range are connected with each other by the rib 5 having a height set within the above range to be hardly separated from each other and increase their strength. Thus, the syringe holder 2 as a whole has an increased strength.

With the configuration enabling the wall parts 4 to be hardly separated from each other and have an increased strength, the deflection of the base 3 can be suppressed and therefore the wall parts 4 are suppressed from tilting, and the deformation of the wall parts 4 due to the deflection of the base 3 can be suppressed so that the openings of the wall parts 4 can be suppressed from being widened. In this way, it is possible to suppress rattling of the syringes 9 in the wall parts 4 according to the aforementioned embodiment. That is, even if the force is applied to the base 3 in such a direction as to deflect the same, the wall parts 4 are hardly separated from each other because the wall parts 4 are connected with each other by the rib 5 having a certain height, and the wall parts 4 are further hardly separated from each other because the wall parts 4 have such an inner diameter as to enable themselves to have increased strength and to be suppressed from being deformed (widening of the openings).

With the wall parts 4 having an inner diameter of 100% or more and 115% or less of the outer diameter of the barrel 91 of the syringe 9, the clearance C between the wall part 4 and the barrel 91 of the syringe 9 is reduced, and hence the syringe 9 hardly rattles (moves) in the wall part 4. Further, with the inner wall surface 41 of the wall part 4 contacting the barrel 91 of the syringe 9 made from the material including polyethylene as the main component, the inner wall surface 41 hardly scratches the syringe 9 made from cyclic polyolefin.

As described above, according to the aforementioned embodiment, it is possible to suppress the deflection of the base 3 and the deformation of the wall part 4 to thereby ensure the strength of the syringe holder 2, and with the syringe holder 2 having the strength thus ensured, to suppress the widening of the clearance C between the wall part 4 and the syringe 9. Therefore, it is possible to suppress rattling of the syringe 9 in the wall part 4. Further, the combination of the configuration for suppressing such rattling and the formation of the inner wall surface 41 of the wall part with material including polyethylene as the main component enables to prevent the syringe 9 from being scratched.

The description will be provided for the case where the syringes 9 are transported while being held by the syringe holder 2 according to the aforementioned embodiment. When no deflection is caused in the base 3, the syringe 9 is transported while being positioned at a substantially center of the wall part. That is, the syringe 9 is transported while a substantially constant clearance C is formed between the inner wall surface 41 of the wall part 4 and the barrel 91 of the syringe 9 in the circumference direction.

In this state, although not avoiding rotation of the syringe 9 in the wall part 4 due to shaking of the syringe storage container 1, impact caused by hitting of the barrel 91 of the syringe 9 against the inner wall surface 41 of the wall part 4 can be alleviated, and hence scratches on the barrel 91 of the syringe 9 can be suppressed. Further, the syringe 9 made from cyclic polyolefin hardly gets scratches because the inner wall surface 41 of the wall part 4 is made from the material including polyethylene as the main component.

As described above, the syringe holder 2 according to this embodiment is a syringe holder 2 for holding the syringe 9 including: a base 3 that is provided with a plurality of insertion through holes 31 through which syringes 9 are inserted; a plurality of wall parts 4 having a cylindrical shape that project respectively from opening end edges 31B of the plurality of insertion through holes 31 of the base 3, the wall parts 4 being formed to have an inner diameter that is the same as a diameter of the insertion through holes 31; and ribs 5, each of which connects each adjacent ones of the plurality of wall parts 4, wherein the ribs 5 have a height from the base 3 of 40% or more and 100% or less of a height from the base3 of the wall part 4, the wall parts 4 have an inner diameter of 100% or more and 115% or less of an outer diameter of a barrel 91 of the syringes 9, and at least an inner wall surface 41 of each of the wall parts 4 are made from a material including polyethylene as a main component.

The syringe storage container 1 according to this embodiment includes the aforementioned syringe holder 2 and a container body 6 that stores the syringe holder 2.

According to the syringe storage container 1 of the aforementioned embodiment, the inner wall surface 41 of each of the wall parts 4 of the syringe holder 2 is made from the material including polyethylene as the main component; each of the ribs 5 connects the wall parts 4 with each other so as to have the height Y from the base 3 of the rib 5 of 40% or more and 100% or less of the height X of the wall parts 4; and the clearance C between the inner diameter of the wall parts 4 and the outer diameter of the barrels 91 of the syringes 9 is adjusted so as to have the inner diameter of the wall parts 4 of 100% or more and 115% or less of the outer diameter of the barrels 91 of the syringes 9. Therefore, it is possible to lower the rigidity of the inner wall surface 41 of the wall parts 4 relative to the conventional one, maintain the shape of the wall parts 4, and thereby keep the clearance C between the inner diameter of the wall parts 4 and the outer diameter of the barrels 91 of the syringes 9 small. Accordingly, the thus configured syringe storage container 1 makes it possible to prevent the syringe 9 from being easily scratched during the transport even if the syringe 9 rubs against the inner wall surface 41 of the wall part 4 and prevent the syringe 9 from easily moving in the wall part 4 to thereby suppress the syringe 9 from rubbing against the inner wall surface 41 of the wall part 4.

Further, with the configuration where the ribs 5 have the height Y of 50.0% or more and 100% or less of the height X of the wall parts 4, each of the ribs 5 connects the wall parts 4 with each other also in an area extending close to the distal end 43 over the center of the wall part 4. Thus, each of the wall parts 4 on the distal end 43 side is hardly widened, and thereby enables to surely keep the clearance C between the wall part 4 and the syringe 9 small.

In the aforementioned embodiment, the inner circumference surfaces 31A of the insertion through holes 31 are configured to increase in diameter toward the rear side 33 of the base 3, and the distal end portions of the inner wall surfaces 41 of the walls 4 are configured to increase in diameter toward the distal end 43. This configuration makes it possible to easily insert the syringe through the wall part 4 and suppress the syringe 9 held therewith from being easily scratched.

Specifically, the distal end portion of the inner wall surface 41 of the wall part 4 is configured to increase in diameter toward the distal end 43 to have the wide opening area of the distal end 43 of the wall part 4, to thereby enable the syringe 9 to be easily inserted through the wall part 4; and the distal end 43 of the wall part 4 and the inner circumference surface 31A of the insertion through hole 31 have increased dimaters to have a small contact area between the barrel 91 of the syringe 9 and the syringe holder 2, so that it is possible to suppress the barrel 91 of the syringe 9 from being scratched.

In the aforementioned embodiment, the inner circumference surface 31A of the insertion through hole 31 is the curved portion 31A1 and continues smoothly without roughness to the inner wall surface 41 of the wall 4 so as not to form an angular portion on the inner circumference surface 31A of the insertion through hole 31 to thereby suppress the barrel 91 of the syringe 9 from being scratched.

In the aforementioned embodiment, the wall parts 4 are aligned in a zigzag alinement (alignment in a hexagonal lattice), adjacent ones of the wall parts 4 are connected with each other by the ribs 5 in six directions at a maximum. Because of this, the strength of the base 3 can be increased.

As described above, according to this embodiment, it is possible to provide the syringe holder 2 that has a sufficient strength and hardly scratches the syringe, and the syringe storage container 1 that includes the syringe holder 2.

The syringe storage container 1 of the present invention is not limited to the aforementioned embodiment. Further, the syringe storage container 1 according to the present invention is not limited to the aforementioned operational effects. Still further, it is a matter of course that various modifications can be made to the syringe storage container 1 according to the present invention without departing from the gist of the present invention.

The aforementioned embodiment was described for the syringe storage container 1 in which the syringe storage container 1 includes the syringe holder 2, but it can be also regarded as the invention for the syringe holder 2. That is, as one of embodiments of the invention according to the syringe holder 2, the syringe holder 2 may include the same configuration as the syringe holder 2 according to the aforementioned embodiment.

The aforementioned embodiment was described by taking, for example, the case where one hundred insertion through holes 31 are formed in the base 3; however, the number and the arrangement of the insertion through holes 31 to be formed in the base are not limited thereto. For example, 8 insertion through holes 31 may be formed along the lateral direction to form one row of 8 holes, and 8 rows may be aligned in the longitudinal direction to form 64 insertion through holes 31. Further, 10 insertion through holes 31 may be formed along the longitudinal direction to form one row of 10 holes, and 16 rows may be aligned in the lateral direction to form 160 insertion through holes 31.

The aforementioned embodiment was described by taking, for example, the case where the wall parts 4 are aligned in a zigzag alinement (alinement in a hexagonal lattice) without limitation thereto. The wall parts 4 may be aligned in a square array.

The aforementioned embodiment was described by taking, for example, the case where the syringe holder 2 made entirely from polyethylene without limitation thereto. In the syringe holder 2, at least the inner wall surface of the wall part 4 may be made from the material including polyethylene as the main component, and the other parts and members such as the base 3, the outer wall surface 42 of the wall part 4, and the rib 5, excluding the inner wall surface 41 of the wall part 4, may be made from other materials.

The aforementioned embodiment was described by taking, for example, the case where the rib 5 connects each directly adjacent ones of the wall parts without limitation thereto. Each rib 5 may connect the wall parts that are not directly adjacent to each other (for example, those located with one row therebetween). For example, each rib 5 may connect with wall part 4 aligned in the 1st row and the wall part 4 aligned in the 3rd row.

### REFERENCE SIGNS LIST

1: Syringe storage container
2: Syringe holder
3: Base
31: Insertion through hole
31A: Inner circumference surface
31A1: Curved portion
31B: Opening end edge
32: Front side
33: Rear side
34: Cutout
35: Extension
4: Wall part
41, S1: Inner wall surface
42: Outer wall surface
43: Distal end
44: Proximal end
5: Rib
51: Proximal end
52: Distal end
6: Container body
61: Bottom part
62: Sidewall
621: Lateral sidewall
622: Longitudinal sidewall
623: Lower sidewall
624: Upper sidewall
624A: Flange
625: Step
9: Syringe
91: Barrel
92: Cap
93: Flange
C: Clearance
P1, P2: Peak
S1: Abutting surface
X: Height of wall part
Y: Height of rib

## Claims

1. A syringe holder (2) for holding a syringe (9) comprising:
a base (3) that is provided with a plurality of insertion through holes (31) through which syringes (9) are inserted;
a plurality of wall parts (4) having a cylindrical shape that project respectively from opening end edges (31B) of the plurality of insertion through holes (31) of the base (3), the wall parts (4) having an inner diameter that is the same as a diameter of the insertion through holes (31);
and ribs (5), each of which connects each adjacent ones of the plurality of wall parts (4),
wherein the ribs (5) have a height from the base (3) of 40% or more and 100% or less of a height from the base (3) of the wall parts (4), the wall parts (4) have an inner diameter of 100% or more and 115% or less of an outer diameter of barrels of the syringes (9), and at least an inner wall surface (41) of each of the wall parts (4) is made from a material including polyethylene as a main component, and
wherein the inner circumference surfaces (31A) of the insertion through holes (31) are configured to increase in diameter toward the rear side (33) of the base (3), and the distal end portions of the inner wall surfaces (41) of the walls are configured to increase in diameter toward the distal end (43),
wherein each wall part (4) has an inner wall surface (41) that constitutes an inner circumference surface of the wall part (4), and an outer wall surface (42) that constitutes an outer circumference surface of the wall part (4),
**characterised in that** the thickness of each wall part (4) decreases from the proximal end (44) toward the distal end (43), and
wherein at each wall part (4), the inner wall surface (41) is formed straight so as to be parallel with the axial direction in a portion other than the distal end portion, while the outer wall surface (42) is formed to incline inward in the radial direction toward the distal end (43).

2. A syringe holder (2) according to Claim 1, wherein the ribs (5) have a height from the base (3) of 50.0% or more and 100% or less of a height from the base (3) of the wall parts (4).

3. A syringe storage container (1) comprising the syringe holder (2) according to Claim 1 or 2 and a container body (6) that stores the syringe holder (2).

## Patentansprüche

1. Spritzenhalter (2) zum Halten einer Spritze (9), umfassend:
eine Basis (3), die mit einer Vielzahl von Einführungsdurchgangslöchern (31) versehen ist, durch die Spritzen (9) eingeführt werden;
eine Vielzahl von Wandteilen (4) mit einer zylindrischen Form, die jeweils von sich öffnenden Endkanten (31B) der Vielzahl von Einführungsdurchgangslöchern (31) der Basis (3) vorstehen, wobei die Wandteile (4) einen Innendurchmesser haben, der der gleiche ist wie ein Durchmesser der Einführungsdurchgangslöcher (31);
und Rippen (5), von denen jede jede benachbarte der Vielzahl von Wandteilen (4) verbindet,
wobei die Rippen (5) eine Höhe von der Basis (3) von 40 % oder mehr und 100 % oder weniger einer Höhe von der Basis (3) der Wandteile (4) aufweisen, die Wandteile (4) einen Innendurchmesser von 100 % oder mehr und 115 % oder weniger eines Außendurchmessers von Zylindern der Spritzen (9) aufweisen, und mindestens eine Innenwandfläche (41) von jedem der Wandteile (4) aus einem Material hergestellt ist, das Polyethylen als eine Hauptkomponente enthält, und
wobei die Innenumfangsflächen (31A) der Einführungsdurchgangslöcher (31) so eingerichtet sind, dass ihr Durchmesser in Richtung der Rückseite (33) der Basis (3) zunimmt, und die distalen Endabschnitte der Innenwandfläche (41) der Wände so eingerichtet sind, dass ihr Durchmesser in Richtung des distalen Endes (43) zunimmt,
wobei jedes Wandteil (4) eine Innenwandfläche (41), die eine Innenumfangsfläche des Wandteils (4) bildet, und eine Außenwandfläche (42), die eine Außenumfangsfläche des Wandteils (4) bildet, aufweist,
**dadurch gekennzeichnet, dass** die Dicke eines jeden Wandteils (4) vom proximalen Ende (44) zum distalen Ende (43) hin abnimmt, und
wobei an jedem Wandteil (4) die Innenwandfläche (41) gerade ausgebildet ist, so dass sie in einem anderen Abschnitt als dem distalen Endabschnitt parallel zur axialen Richtung verläuft, während die Außenwandfläche (42) so ausgebildet ist, dass sie in der radialen Richtung zum distalen Ende (43) hin nach innen geneigt ist.

2. Spritzenhalter (2) nach Anspruch 1, wobei die Rippen (5) eine Höhe von der Basis (3) von 50,0% oder mehr und 100% oder weniger einer Höhe von der Basis (3) der Wandteile (4) aufweisen.

3. Spritzenaufbewahrungsbehälter (1), umfassend den Spritzenhalter (2) nach Anspruch 1 oder 2 und einen Behälterkörper (6), der den Spritzenhalter (2) aufnimmt.

## Revendications

1. Support de seringue (2) pour tenir une seringue (9) comprenant :
une base (3) qui est pourvue d'une pluralité de trous traversants d'insertion (31) à travers lesquels les seringues (9) sont insérées ;
une pluralité de parties de paroi (4) ayant une forme cylindrique qui font saillie respectivement à partir de bords d'extrémité d'ouverture (31B) de la pluralité de trous traversants d'insertion (31) de la base (3), les parties de paroi (4) ayant un diamètre intérieur qui est le même qu'un diamètre des trous traversants d'insertion (31) ;
et des nervures (5), chacune d'entre elles reliant chacune des parties adjacentes de la pluralité de parties de paroi (4),
dans lequel les nervures (5) ont une hauteur à partir de la base (3) de 40 % ou plus et de 100 % ou moins d'une hauteur à partir de la base (3) des parties de paroi (4), les parties de paroi (4) ont un diamètre intérieur de 100 % ou plus et de 115 % ou moins d'un diamètre extérieur de corps des seringues (9), et au moins une surface de paroi intérieure (41) de chacune des parties de paroi (4) est faite d'un matériau comprenant du polyéthylène comme composant principal, et
dans lequel les surfaces de circonférence intérieure (31A) des trous traversants d'insertion (31) sont configurées pour augmenter en diamètre vers le côté arrière (33) de la base (3), et les portions d'extrémité distale des surfaces de paroi intérieures (41) des parois sont configurées pour augmenter en diamètre vers l'extrémité distale (43),
dans lequel chaque partie de paroi (4) a une surface de paroi intérieure (41) qui constitue une surface de circonférence intérieure de la partie de paroi (4), et une surface de paroi extérieure (42) qui constitue une surface de circonférence extérieure de la partie de paroi (4),
**caractérisé en ce que**
l'épaisseur de chaque partie de paroi (4) diminue de l'extrémité proximale (44) vers l'extrémité distale (43), et
dans lequel, au niveau de chaque partie de paroi (4), la surface de paroi intérieure (41) est formée de manière rectiligne de façon à être parallèle à la direction axiale dans une portion autre que la portion d'extrémité distale, tandis que la surface de paroi extérieure (42) est formée pour s'incliner vers l'intérieur dans la direction radiale vers l'extrémité distale (43).

2. Support de seringue (2) selon la revendication 1, dans lequel les nervures (5) ont une hauteur à partir de la base (3) de 50,0 % ou plus et de 100 % ou moins d'une hauteur à partir de la base (3) des parties de paroi (4).

3. Récipient de stockage de seringues (1) comprenant le support de seringue (2) selon la revendication 1 ou 2 et un corps de récipient (6) qui stocke le support de seringue (2) .
